# EUROPÄISCHE PATENTANMELDUNG

(11) **EP 3 278 860 A1**
(43) Veröffentlichungstag der Anmeldung: **07.02.2018**
(21) Anmeldenummer: 16182257.2
(22) Anmeldetag: 01.08.2016
(51) Int. Cl.: B01D 53/04, B01D 53/047, C07C 2/82, C07C 2/84, C07C 7/12

(54) **PROZESS ZUR ERZEUGUNG UND VERARBEITUNG VON PRODUKTEN DER OXIDATIVEN KOPPLUNG VON METHAN UND ENTSPRECHENDE ANLAGE**

(71) Anmelder: Linde Aktiengesellschaft, 80331 München (DE)
(72) Erfinder: Voss, Christian, 82538 Geretsried (DE); Leitmayr, Werner, 86633 Neuburg/Donau (DE); Kemper, Rainer, 81375 München (DE); Mündl, Florian, 83624 Otterfing (DE); Haidegger, Ernst, 85521 Riemerling (DE)
(74) Vertreter: m patent group

(57) **Zusammenfassung**

Die Erfindung schlägt einen Prozess (100, 200) zur Erzeugung und Verarbeitung von Produkten der oxidativen Kopplung von Methan, vor, bei dem unter Verwendung eines Verfahrens zur oxidativen Kopplung von Methan (1) ein Gasgemisch bereitgestellt wird, das zumindest Ethylen, Ethan und Methan sowie niedriger als Methan und höher als Ethan siedende Verbindungen enthält. Es ist vorgesehen, dass aus dem Gasgemisch unter Verwendung einer Druckwechseladsorption (4) ein Teil des Methans und zumindest ein Teil der niedriger als Methan siedenden Komponenten entfernt werden, und dass das Gasgemisch anschließend weiteren Aufbereitungsschritten unterworfen wird, wobei die Druckwechseladsorption (4) die Verwendung eines unteratmosphärischen Desorptionsdruckniveaus umfasst. Eine entsprechende Anlage ist ebenfalls Gegenstand der vorliegenden Erfindung.

## Beschreibung

Die Erfindung betrifft einen Prozess zur Erzeugung und Verarbeitung von Produkten der oxidativen Kopplung von Methan, sowie eine entsprechende Anlage gemäß den jeweiligen Oberbegriffen der unabhängigen Patentansprüche.

### Stand der Technik

Die oxidative Kopplung von Methan ist in der Literatur beschrieben, beispielsweise bei J.D. Idol et al., "Natural Gas", in: J.A. Kent (Hrsg.), "Handbook of Industrial Chemistry and Biotechnology", Band 2, 12. Auflage, Springer, New York 2012.

Die oxidative Kopplung von Methan umfasst nach derzeitigem Kenntnisstand eine katalysierte Gasphasenreaktion von Methan mit Sauerstoff, bei der von zwei Methanmolekülen jeweils ein Wasserstoffatom abgespalten wird. Die dabei entstehenden Methylradikale reagieren zunächst zu einem Ethanmolekül. Bei der Reaktion wird ferner ein Wassermolekül gebildet. Bei geeigneten Verhältnissen von Methan zu Sauerstoff, geeigneten Reaktionstemperaturen und der Wahl geeigneter Katalysebedingungen erfolgt anschließend eine Oxydehydrierung des Ethans zu Ethylen, einer Zielverbindung bei der oxidativen Kopplung von Methan. Hierbei wird ein weiteres Wassermolekül gebildet.

Die Reaktionsbedingungen bei der oxidativen Kopplung von Methan umfassen klassischerweise eine Temperatur von 500 bis 900 °C und einen Druck von 1 bis 10 bar sowie hohe Raumgeschwindigkeiten. Jüngere Entwicklungen gehen insbesondere auch in Richtung der Verwendung tieferer Temperaturen. Die Reaktion kann homogen- und heterogenkatalytisch im Festbett oder in der Wirbelschicht erfolgen. Bei der oxidativen Kopplung von Methan können auch höhere Kohlenwasserstoffe mit bis zu sechs oder acht Kohlenstoffatomen gebildet werden; der Schwerpunkt liegt jedoch auf Ethan bzw. Ethylen und ggf. noch Propan bzw. Propylen.

Insbesondere aufgrund der hohen Bindungsenergie zwischen Kohlenstoff und Wasserstoff im Methanmolekül sind die Ausbeuten bei der oxidativen Kopplung von Methan vergleichsweise gering. Außerdem begünstigen die vergleichsweise harschen Reaktionsbedingungen und Temperaturen, die zur Spaltung dieser Bindungen erforderlich sind, auch die weitere Oxidation der Methylradikale und anderer Intermediate zu Kohlenmonoxid und Kohlendioxid.

Wenngleich den geringen Ausbeuten und der Bildung von Kohlenmonoxid und Kohlendioxid teilweise durch die Wahl optimierter Katalysatoren und angepasster Reaktionsbedingungen entgegengewirkt werden kann, enthält ein bei der oxidativen Kopplung von Methan gebildetes Gasgemisch neben den Zielverbindungen wie Ethylen und ggf. Propylen beträchtliche Mengen nicht umgesetzten Methans sowie Wasser, Kohlenmonoxid und Kohlendioxid. Ein derartiges Gasgemisch wird nachfolgend auch als "Produktgemisch" der oxidativen Kopplung von Methan bezeichnet, obwohl es nicht nur die gewünschten Produkte, sondern auch nicht umgesetzte Edukte und Nebenprodukte enthält.

Bei der oxidativen Kopplung von Methan können Reaktoren eingesetzt werden, in denen einer katalytischen Zone eine nichtkatalytische Zone nachgeschaltet ist. Das in der katalytischen Zone gebildete Produktgemisch wird in die nichtkatalytische Zone überführt, wo es zunächst noch auf den vergleichsweise hohen Temperaturen vorliegt, die in der katalytischen Zone eingesetzt werden. Insbesondere durch die Anwesenheit des bei der oxidativen Kopplung von Methan gebildeten Wassers ähneln die Reaktionsbedingungen hier jenen herkömmlicher Dampfspaltverfahren (engl. Steam Cracking). Daher können hier Ethan und höhere Paraffine zu Olefinen umgesetzt werden. In die nichtkatalytische Zone können auch weitere Paraffine eingespeist werden, so dass die Restwärme der oxidativen Kopplung von Methan in besonders vorteilhafter Weise ausgenutzt werden kann. Ein derartiges Dampfspalten in einer der katalytischen Zone nachgeschalteten nichtkatalytischen Zone wird auch als "Post Bed Cracking" bezeichnet. Nachfolgend wird hierfür auch der Begriff "postkatalytisches Dampfspalten" verwendet.

Bezüglich den beim Dampfspalten herrschenden Reaktionsbedingungen sei ebenfalls auf Fachliteratur wie den Artikel "Ethylene" in Ullmann's Encyclopedia of Industrial Chemistry, Onlineausgabe, 15. April 2007, DOI 10.1002/14356007.a10_045.pub2, verwiesen. Das Dampfspalten wird beispielsweise zur Gewinnung von kurzkettigen Olefinen wie Ethylen und Propylen, Diolefinen wie Butadien oder von Aromaten eingesetzt, ist jedoch nicht hierauf beschränkt.

Ist nachfolgend von einem "Reaktor, der zur oxidativen Kopplung von Methan eingerichtet ist", die Rede, soll hierunter ein Reaktor verstanden werden, der zumindest in einer Reaktorzone einen Katalysator aufweist (beispielsweise in Form eines Festbett- oder Wirbelschichtkatalysators), welcher sich für die oxidative Kopplung von Methan eignet. Zumindest in dieser Reaktorzone können durch geeignete Mittel, beispielsweise durch Brenner und vorgeschaltete Verdichter, Temperatur- und Druckbedingungen geschaffen werden, die unter Einfluss des Katalysators zu einer oxidativen Kopplung von Methan führen, wie zuvor erläutert. Ein entsprechender Reaktor kann neben der erläuterten Reaktorzone weitere Reaktorzonen aufweisen, beispielsweise eine nichtkatalytische Zone, die zum oben erläuterten postkatalytischen Dampfspalten ("Post Bed Cracking") verwendet wird.

In entsprechender Weise soll, wenn nachfolgend davon die Rede ist, dass "unter Verwendung eines Verfahrens zur oxidativen Kopplung von Methan" ein Produktgemisch gebildet wird, hierunter verstanden werden, dass hierbei ein Verfahren beteiligt ist, das die zuvor erläuterten Reaktionen umfasst, insbesondere die Bildung von Methylradikalen, deren Kopplung zu Ethan und die anschließende Oxydehydrierung. Zusätzlich können jedoch weitere Verfahren bzw. Verfahrensschritte umfasst sein, insbesondere das oben erläuterte postkatalytische Dampfspalten ("Post Bed Cracking"). Diesem oder diesen weiteren Verfahren oder Verfahrensschritten können weitere Einsätze zugeführt werden, die zumindest teilweise zu Produkten umgesetzt werden, welche sich in dem Produktgemisch wiederfinden. Mit anderen Worten muss bei der Bildung des Produktgemischs, die "unter Verwendung" des Verfahrens zur oxidativen Kopplung von Methan erfolgt, nicht ausschließlich die oxidative Kopplung von Methan beteiligt sein.

Produktgemische, die unter Verwendung eines Verfahrens zur oxidativen Kopplung von Methan gebildet werden, enthalten aus den oben erläuterten Gründen insbesondere große Mengen an Methan, das vorteilhafterweise abgetrennt und in die oxidative Kopplung zurückgeführt werden sollte. Die trenntechnische Bearbeitung eines entsprechenden Produktgemischs stellt sich dabei aufgrund des hohen Methananteils bei dem vergleichsweise geringem Gehalt an Zielprodukten als ausgesprochen aufwendig dar.

Die vorliegende Erfindung will daher einen Prozess bereitstellen, bei dem insbesondere die Bearbeitung eines entsprechenden Produktgemischs gegenüber dem Stand der Technik verbessert ist.

### Offenbarung der Erfindung

Vor diesem Hintergrund schlägt die vorliegende Erfindung einen Prozess zur Erzeugung und Verarbeitung von Produkten der oxidativen Kopplung von Methan, und eine entsprechende Anlage mit den Merkmalen der unabhängigen Patentansprüche vor. Ausgestaltungen sind jeweils Gegenstand der abhängigen Patentansprüche sowie der nachfolgenden Beschreibung.

Vor der Erläuterung der Merkmale und Vorteile der vorliegenden Erfindung werden noch einige Grundlagen und die verwendeten Begriffe erläutert.

Ist nachfolgend von, im Vergleich zu einer Verbindung, "höher siedenden" oder "tiefer siedenden" bzw. "niedriger siedenden" Verbindungen die Rede, handelt es sich hierbei um Verbindungen, die einen höheren bzw. niedrigeren Siedepunkt aufweisen als jene Verbindung, mit der diese Verbindungen verglichen werden. Beispielsweise sind Kohlenmonoxid, Wasserstoff, Stickstoff und Argon Verbindungen, die niedriger sieden als Methan. Acetylen und Kohlenwasserstoffe mit drei und mehr Kohlenstoffatomen sind hingegen Verbindungen, die höher sieden als Ethan.

Flüssige und gasförmige Gemische können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 10%, 5%, 1%, 0,1% oder 0,01% auf molarer, Gewichts- oder Volumenbasis stehen kann. Der Begriff "überwiegend" kann der Definition von "reich" entsprechen. Flüssige und gasförmige Gemische können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Das flüssige oder gasförmige Gemisch ist "angereichert", wenn es zumindest den 1,1-fachen, 1,5-fachen, 2-fachen, 5-fachen, 10-fachen, 100-fachen oder 1.000-fachen Gehalt, "abgereichert", wenn es höchstens den 0,9-fachen, 0,5-fachen, 0,1-fachen, 0,01-fachen oder 0,001-fachen Gehalt einer entsprechenden Komponente, bezogen auf das Ausgangsgemisch, enthält. Ist vorliegend beispielsweise von "Methan", "Wasserstoff" oder "Ethylen" die Rede, sei darunter auch ein Gemisch verstanden, das reich an der entsprechenden Komponente ist. Es kann sich jedoch auch um das jeweilige Reingas handeln.

Ein flüssiges oder gasförmiges Gemisch ist von einem anderen flüssigen oder gasförmigen Gemisch (auch als Ausgangsgemisch bezeichnet) "abgeleitet" oder aus diesem Gemisch oder unter Verwendung dieses Gemischs "gebildet", wenn es zumindest einige in dem Ausgangsgemisch enthaltene oder aus diesem erhaltene Komponenten aufweist. Ein in diesem Sinne gebildetes Gemisch kann aus dem Ausgangsgemisch durch Abtrennen oder Abzweigen eines Teilstroms oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen gebildet werden. Ein "Bilden", beispielsweise eines Einsatzgemischs für einen anschließenden Trennprozess, kann jedoch auch einfach das Führen eines entsprechenden Gemischs in einer geeigneten Leitung und ein Zuführen zu dem Trennprozess darstellen.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste ein. Entsprechendes gilt für Temperaturniveaus. Bei dem hier in bar angegebenen Druckniveaus handelt es sich um Absolutdrücke.

### Vorteile der Erfindung

Die vorliegende Erfindung geht von der Erkenntnis aus, dass es besonders vorteilhaft ist, ein in einem Verfahren zur oxidativen Kopplung von Methan gebildetes Produktgemisch, das, wie zuvor erläutert, ausgesprochen reich an Methan ist und neben den Zielprodukten, insbesondere Ethylen, Nebenprodukte der oxidativen Kopplung von Methan enthält, zunächst einer nachfolgend als "unscharf" bezeichneten Entfernung von Methan mittels einer Druckwechseladsorption zu unterwerfen. Bei einer derartigen "unscharfen" Entfernung von Methan wird Methan nicht vollständig entfernt, sondern es verbleibt ein Rest in dem Produktgemisch, der im Rahmen einer anschließenden trenntechnischen Bearbeitung des Produktgemischs, insbesondere in einem Demethanizer bekannter oder an den Prozess angepasster Art, abgetrennt wird. Durch die Abreicherung des Produktgemischs an Methan sinkt jedoch der Aufwand für diese anschließende trenntechnische Bearbeitung deutlich. Weil bei der "unscharfen" Entfernung von Methan dieses nicht vollständig entfernt werden muss, kann die dazu verwendete Druckwechseladsorption vergleichsweise einfach und kostengünstig mit nur geringem apparativem Aufwand durchgeführt werden.

Die besonderen Vorteile der vorliegenden Erfindung ergeben sich daraus, dass die Druckwechseladsorption die Verwendung eines unteratmosphärischen Desorptionsdruckniveaus umfasst. Es kommt also eine sogenannte Vakuum-Druckwechseladsorption (engl. Vacuum Pressure Swing Adsorption, VPSA) zum Einsatz. Im Rahmen der vorliegenden Erfindung wird, wie auch nachfolgend noch angegeben, das zu bearbeitende Gasgemisch der Druckwechseladsorption auf einem deutlich überatmosphärischen Druckniveau zugeführt, das hier auch als Adsorptionsdruckniveau bezeichnet wird.

Das Gasgemisch wird auf dem Adsorptionsdruckniveau in einen oder mehrere Behälter eingeleitet, der oder die ein geeignetes Adsorptionsmaterial aufweisen. Im Rahmen der vorliegenden Erfindung, wo die Abreicherung an Methan und niedriger als Methan siedender Verbindungen der Hauptzweck ist, wird als Adsorptionsmaterial ein Schichtbett, vorzugsweise aus einer bis drei Schichten von Adsorbentien mit Alugel und Silikagel, verwendet. Besonders vorteilhaft ist dabei ein Bettaufbau, bei dem als oberste Schicht Alugel eingesetzt wird. Dabei wird der Adsorber während der Adsorption von unten nach oben durchströmt. An dieses werden überwiegend höher als Methan siedende Verbindungen bei und nach der Einspeisung während eines Zeitraums adsorbiert, der hier als Adsorptionsphase bezeichnet wird. Das Methan und die niedriger als Methan siedenden Verbindungen passieren hingegen den oder die Behälter während der Adsorptionsphase auf dem Adsorptionsdruckniveau.

Zur Desorption der adsorbierten Komponenten erfolgt bei einer Druckwechseladsorption in einem Zeitraum nach der Adsorptionsphase, der hier als Desorptionsphase bezeichnet wird, eine Druckabsenkung auf ein geringeres Druckniveau, das hier als Desorptionsdruckniveau bezeichnet wird. Bei diesem Desorptionsdruckniveau desorbieren während der Desorptionsphase die zuvor adsorbierten Komponenten. Die Druckabsenkung erfolgt im Rahmen der vorliegenden Erfindung auf ein unteratmosphärisches Desorptionsdruckniveau. Die Verwendung eines unteratmosphärischen Desorptionsdruckniveaus erlaubt eine Verbesserung der Leistungsfähigkeit der Druckwechseladsorption insgesamt.

Durch die Desorption auf dem unteratmosphärischen Desorptionsdruckniveau kann die Restbeladung mit unerwünschten Komponenten deutlich verringert werden. Hieraus resultiert eine hohe Gastrennkapazität, die zu besserer Auschleusung an Methan und leichter siedenden Komponenten führt. Zudem wird ein üblicher, jedoch nicht zwingend erforderlicher Spülgasbedarf deutlich reduziert. Dies führt zu einer minimierten Kontamination der in der Druckwechseladsorption erhaltenen schwerer siedenden Fraktion durch Spülgaskomponenten, z.B. Methan.

Durch das unteratmosphärische Druckniveau bei der Desorption vergrößert sich das Druckverhältnis der nachfolgenden Verdichtung der schwerer siedenden Fraktion, jedoch verringert sich der durch die erhöhte Ausschleusung der leichter siedenden Fraktion, z.B. Methan, zu verdichtende Mengenstrom. Daher ist der Energiebedarf der Verdichtung im Vergleich zur überatmosphärischen Desorption geringer oder höchstens gleich. Zusammen mit dem verringerten apparativen Aufwand erzielt die vorliegende Erfindung daher beträchtliche Vorteile.

Insgesamt schlägt die vorliegende Erfindung einen Prozess zur Erzeugung und Verarbeitung von Produkten der oxidativen Kopplung von Methan, vor, bei dem unter Verwendung eines Verfahrens zur oxidativen Kopplung von Methan ein Gasgemisch bereitgestellt wird, das zumindest Ethylen, Ethan und Methan sowie niedriger als Methan und höher als Ethan siedende Verbindungen enthält. Wie auch zuvor erläutert, können im Fall der oxidativen Kopplung von Methan entsprechende Reaktoren zum Einsatz kommen. Insbesondere können auch mehrere solcher Reaktoren parallel angeordnet sein, d.h. mehrere entsprechender Gasgemische können parallel zueinander bereitgestellt werden. Ist nachfolgend von einem Gasgemisch die Rede, beziehen sich die jeweiligen Erläuterungen auch auf derartige Fälle.

Erfindungsgemäß ist vorgesehen, dass aus dem Gasgemisch unter Verwendung einer Druckwechseladsorption ein Teil des Methans und zumindest ein Teil der niedriger als Methan siedenden Komponenten entfernt werden, und dass das Gasgemisch anschließend weiteren Aufbereitungsschritten unterworfen wird, wobei die Druckwechseladsorption die Verwendung eines unteratmosphärischen Desorptionsdruckniveaus umfasst. Bei einer parallelen Bereitstellung mehrerer Gasgemische können auch entsprechende Druckwechseladsorptionsschritte bzw. Druckwechseladsorptionseinrichtungen parallel vorgesehen sein. Auch hier gelten die nachfolgenden Erläuterungen entsprechend. Vorteile dieses Vorgehens, d.h. einer Parallelisierung, werden auch nachfolgend noch erläutert.

Der besondere Vorteil bei der Verwendung eines unteratmosphärischen Desorptionsdruckniveaus liegt in der deutlich verbesserten Abreicherung an Methan. aus den oben erläuterten Gründen. Auf diese Weise kann sichergestellt werden, dass trotz der anfänglich ausgesprochen hohen Gehalte an Methan in einem entsprechenden Gasgemisch die stromab zur weiteren Bearbeitung verwendeten Einrichtungen kleiner und kostengünstiger dimensioniert werden können.

Dies betrifft insbesondere den "kalten Teil" einer entsprechenden Trennanlage, in dem eine Tieftemperaturtrennung vorgenommen wird, die sich der Druckwechseladsorption anschließt. In diesem kalten Teil können aufgrund der im Rahmen der vorliegenden Erfindung geringeren zu bearbeitenden Volumina kostenintensive, tieftemperaturbeständige Materialien eingespart werden. Dies betrifft in entsprechender Weise auch die bei einer entsprechenden Tieftemperaturtrennung eingesetzten Kältemittel, erforderliche Verdichterleistungen usw. Würde keine Methanabreicherung vorgenommen, wären die Baubarkeitsgrenzen in einer entsprechenden Tieftemperaturtrennung ggf. aufgrund der hohen zu bearbeitenden Methanmengen rasch erreicht bzw. würde die Baubarkeit in der Tieftemperaturtrennung die stromaufwärtigen Verfahrensschritte limitieren. In diesem Fall wäre eine Parallelisierung der Tieftemperaturtrennung, die jedoch mit entsprechendem Kostenaufwand verbunden ist und ggf. die Wirtschaftlichkeit des Gesamtprozesses in Frage stellen würde, erforderlich.

Die vorliegende Erfindung ermöglicht es dagegen, ggf. sogar den umgekehrten Weg einer Parallelisierung der oxidativen Kopplung von Methan, zu gehen, und damit eine einzige Tieftemperaturtrennung für mehrere entsprechender Synthesestränge bereitzustellen. Aufgrund der vergleichsweise geringen Gehalte an höher als Methan siedenden Verbindungen ist der zur Trennung dieser Komponenten aufzuwendende apparative Aufwand vergleichsweise gering.

Im Rahmen der vorliegenden Erfindung liegt das Desorptionsdruckniveau vorteilhafterweise bei 200 bis 800 mbar, insbesondere bei 350 bis 500 mbar. Es können jedoch auch höhere und niedrigere unteratmosphärische Desorptionsdruckniveaus eingesetzt werden, sofern sich bei diesen eine ausreichende Methanabreicherung bzw. Aufkonzentrierung schwererer Komponenten des Gasgemischs ergibt. Ein "unteratmosphärisches" Druckniveau liegt unterhalb des jeweiligen Atmosphärendrucks, insbesondere unterhalb von 1 bar, vorzugsweise mindestens 10 mbar, 20 mbar, 30 mbar, 40 mbar, 50 mbar, 100 mbar, 200 mbar, 300 mbar, 400 mbar, 500 mbar, 600 mbar, 700 mbar oder 800 mbar unterhalb.

Vorteilhafterweise wird in dem erfindungsgemäßen Prozess in der Druckwechseladsorption ein Adsorptionsdruckniveau von 10 bis 30 bar, insbesondere von 13 bis 17 bar, verwendet. Bei einem entsprechenden Adsorptionsdruckniveau ergibt sich eine ausreichend gute und vollständige Adsorption der höher als Methan siedenden Verbindungen an das verwendete Adsorptionsmaterial und eine besonders gute Kompatibilität mit weiteren Verfahrensschritten. Eine weitere Erhöhung des Adsorptionsdruckes führt zu einer lediglich geringen Anstieg der Adsorptionsbeladung bei gleichzeitig erheblich höheren Investitionskosten sowie Energieaufwand für die Verdichtung des Produktgemisches.

Im Rahmen der vorliegenden Erfindung können unter Verwendung der Druckwechseladsorption aus dem Gasgemisch 70 bis 90 Molprozent, insbesondere 80 bis 85 Molprozent, des Methans entfernt werden. Gegenüber einer Druckwechseladsorption unter Verwendung eines überatmosphärischen Desorptionsdruckniveaus oder eines Desorptionsdruckniveaus bei Atmosphärendruck entspricht dies einer Steigerung der Abreicherung von Methan um 10 bis 30 Prozent. Diese Effizienzsteigerung ist im Hinblick auf die hohen Methangehalte von Gasgemischen aus der oxidativen Kopplung von Methan zu sehen, die um 70 bis 80 Molprozent liegt. Eine entsprechende Steigerung kann hierbei ohne weiteres zu einer Verringerung des stromab der Druckwechseladsorption zu bearbeitenden Restgases um den Faktor zwei führen.

Wie bereits zuvor erwähnt, kann im Rahmen der vorliegenden Erfindung insbesondere eine Parallelisierung der oxidativen Kopplung von Methan von Vorteil sein. Diese erfolgt zumindest bis und einschließlich der Druckwechseladsorption. Auch ein Teil der sich anschließenden weiteren Aufbereitungsschritte kann noch parallel durchgeführt werden. Insbesondere stromab einer Druckwechseladsorptionsanlage inklusive der sich anschließenden Tieftemperaturtrennung werden vorzugsweise die separaten Gasströme jedoch vereinigt und die sich anschließenden Trennschritte gemeinsam durchgeführt. Mit anderen Worten werden die Prozessgruppen bzw. Verarbeitungsschritte stromab der Druckwechseladsorptionsanlage inklusive der Tieftemperaturtrennung mit den parallel erzeugten und bis zu einem gewissen Grad aufbereiteten Gasgemischen gespeist.

Vorteilhaft ist also, mit anderen Worten, insbesondere ein Prozess, bei dem unter Verwendung mehrerer Einrichtungen, die zur Durchführung der oxidativen Kopplung von Methan eingerichtet sind, jeweils ein Gasgemisch bereitgestellt wird, das zumindest Ethylen, Ethan und Methan sowie niedriger als Methan und höher als Ethan siedende Verbindungen enthält, wobei aus den Gasgemischen parallel zueinander in wenigstens zwei Einrichtungen, die zur Druckwechseladsorption eingerichtet sind, ein Teil des Methans und zumindest ein Teil der niedriger als Methan siedenden Komponenten entfernt werden, und wobei die Gasgemische anschließend vereinigt und zumindest einem der weiteren Aufbereitungsschritte unterworfen werden.

Vorteile ergeben sich in einem entsprechenden Verfahren, wie bereits erwähnt, insbesondere, wenn die weiteren Aufbereitungsschritte komplexe Prozessgruppen wie eine Tieftemperaturtrennung umfassen. Bei Verfahren zur oxidativen Kopplung von Methan kann ab einer bestimmten Anlagenkapazität durch die Parallelisierung bis und einschließlich der Druckwechseladsorption eine wirtschaftlich sinnvolle Baubarkeitsgrenze eingehalten werden, bei gleichzeitig einstrangiger Ausführung der stromab der Druckwechseladsorption liegenden Prozessgruppen inklusive der Tieftemperaturtrennung.

Mit besonderem Vorteil umfasst die Tieftemperaturtrennung, falls vorgesehen, eine Demethanisierung, der zumindest ein Teil des Gasgemischs bzw. ein aus diesem gebildetes Aufbereitungsprodukt als Trenneinsatz unterworfen wird. Die Demethanisierung kann dabei insbesondere als sogenannte "Front End"-Demethanisierung vorgesehen sein, d.h. es erfolgt vorab keine Abtrennung von beispielsweise Kohlenwasserstoffen mit zwei oder drei Kohlenstoffatomen, also keine Deethanisierung oder Depropanisierung. Mit anderen Worten kann im Rahmen der vorliegenden Erfindung ein "Demethanizer First"-Verfahren eingesetzt werden. Zu Details entsprechender Verfahren sei auf den eingangs zitierten Artikel "Ethylene" in Ullmann's Encyclopedia of Industrial Chemistry verwiesen. Ein derartiges "Demethanizer First"-Verfahren ist im Rahmen der vorliegenden Erfindung besonders vorteilhaft, weil typische Produktgemische aus der oxidativen Kopplung von Methan, typischerweise (z.B. im Vergleich zu Produktgemischen aus Dampfspaltverfahren) vergleichsweise geringe Mengen an höher als Ethan siedenden Verbindungen enthalten. Dies werden daher vorzugsweise stromab der Demethanisierung abgetrennt, um die nachgeordneten Trenneinrichtungen aus den erwähnten Gründen kleiner dimensionieren zu können.

Der Demethanisierung können sich in einer entsprechenden Tieftemperaturtrennung weitere bekannte Verfahrensschritte eingesetzt werden, die sich nach den Gehalten an höheren Kohlenwasserstoffen richten. Beispielsweise können diese eine Deethanisierung, Depropanisierung, C2-Trennung, C3-Trennung usw. umfassen. Für Details sei auf die zitierte Fachliteratur verwiesen.

Im Rahmen der vorliegenden Erfindung kann auch vorgesehen sein, dass die weiteren Aufbereitungsschritte wenigstens einen absorptiven Schritt umfassen. Beispielsweise kann im Rahmen der vorliegenden Erfindung eine absorptive Demethanisierung eingesetzt werden. Bei dieser werden zunächst ein Teil des Methans sowie der überwiegende Teil des Ethans und des Ethylens und der höher als Ethan siedenden Verbindungen mittels einer Absorptionsflüssigkeit bzw. Waschflüssigkeit aus dem ausgewaschen. Die Absorptionsflüssigkeit enthält dabei Kohlenwasserstoffe mit zumindest drei oder vier Kohlenstoffatomen, beispielsweise Kohlenwasserstoffe mit drei und/oder Kohlenwasserstoffe mit vier und/oder Kohlenwasserstoffe mit fünf und/oder Kohlenwasserstoffe mit sechs Kohlenstoffatomen. Insbesondere kann es sich um Butan oder ein Gemisch von Kohlenwasserstoffen mit vier Kohlenstoffatomen handeln. Die absorptive Demethanisierung stellt eine Alternative zur "klassischen", d.h. destillativen, Demethanisierung dar, wie sie beispielsweise aus dem Bereich der Dampfspaltverfahren bekannt und dort üblich ist. Verfahren zur absorptiven Demethanisierung, die auch im Rahmen der vorliegenden Erfindung einsetzbar sind, sind grundsätzlich bekannt, beispielsweise aus der EP 0 675 094 A2.

Insbesondere in Kombination mit der Abreicherung an Methan durch Druckwechseladsorption bietet der Einsatz eines absorptiven Verfahrens der Demethanisierung Vorteile. Bei einer absorptiven Demethanisierung wäre zur Bearbeitung des zu demethanisierenden Produktgemischs ohne eine Abreicherung an Methan mehr als das Zehnfache an Absorptionsflüssigkeit, beispielsweise Butan, erforderlich. Würde ein mittels oxidativer Kopplung von Methan gewonnenes Produktgemisch daher unmittelbar mit in einem absorptiven Demethanizer behandelt, wären daher sehr große Mengen an Absorptionsflüssigkeit erforderlich. Durch die Abreicherung mittels Druckwechseladsorption im Rahmen der vorliegenden Erfindung kann diese Menge verringert werden, beispielsweise auf das Drei- bis Vierfache des zu bearbeitenden Produktgemischs.

Eine absorptive Demethanisierung hat den besonderen Vorteil, dass hierbei zur Abtrennung von Methan und ggf. niedriger als Methan siedender Verbindungen deutlich höhere Temperaturen zum Einsatz kommen können als sie in überwiegend oder ausschließlich destillativen Verfahren möglich sind. In einem überwiegend oder ausschließlich destillativen Verfahren müssen Temperaturen von -100 °C und weniger zur Abtrennung von Methan und ggf. Wasserstoff zum Einsatz kommen, was die Verwendung von Ethylen als Kältemittel erfordert. In der Regel ist dabei zusätzlich der Einsatz von Turboexpandern zur Erzielung von Temperaturen von -130 °C und weniger erforderlich. Ein absorptives Verfahren schafft hier Abhilfe und ermöglicht den Verzicht auf den Einsatz entsprechend niedriger Temperaturen und unter anderem den damit verbundenen Einsatz kostenintensiver Materialien.

Weitere Aufbereitungsschritte stromab der Druckwechseladsorption und insbesondere stromauf einer Tieftemperaturtrennung können vorgesehen sein. In dem erfindungsgemäßen Prozess kann das Gasgemisch insbesondere Kohlendioxid enthalten. Der oder die weiteren Aufbereitungsschritte umfassen daher insbesondere eine Sauergasentfernung bzw. Kohlendioxidentfernung. Vorteilhafterweise wird das Gasgemisch dabei stromab der Druckwechseladsorption verdichtet und im Zuge oder nach dieser Verdichtung der Sauergasentfernung unterworfen. Sauergasentfernungsschritte, die insbesondere zur Abtrennung von Kohlendioxid und ggf. anderer Verbindungen wie Schwefelwasserstoff dienen, sind beispielsweise aus dem Bereich der Dampfspaltverfahren bekannt.

Eine Sauergasentfernung, wie sie auch im Rahmen der vorliegenden Erfindung zum Einsatz kommen kann, kann insbesondere eine Aminwäsche umfassen. Bei einer derartigen Aminwäsche wird ein entsprechendes Gasgemisch mit einer aminhaltigen Waschlösung (Waschlauge) in Kontakt gebracht, mittels derer insbesondere Kohlendioxid und andere Sauergase aus dem Gasgemisch ausgewaschen werden. Bei der Sauergasentfernung abgetrenntes Kohlendioxid kann, wie oben erwähnt, teilweise oder vollständig bei einer Methanisierung eingesetzt werden, insbesondere wenn dort eine überstöchiometrische Wasserstoffmenge vorliegt.

In dem erfindungsgemäßen Prozess kann, wie erwähnt, das Gasgemisch insbesondere Wasser enthalten. In diesem Fall umfassen der oder die weiteren Aufbereitungsschritte insbesondere eine Trocknung des Produktgemischs. Vorteilhafterweise wird im Rahmen der Erfindung das Produktgemisch insbesondere nach der Sauergasentfernung einer derartigen Trocknung unterworfen.

In dem erfindungsgemäßen Prozess können die die höher als Ethan siedenden Verbindungen in dem Gasgemisch Acetylene und/oder Diolefine umfassen. Daher ist es von besonderem Vorteil, wenn der oder die weiteren Aufbereitungsschritte eine Hydrierung der Acetylene und/oder der Diolefine zu den entsprechenden (Mono-) Olefinen umfassen. Eine derartige Hydrierung ist von besonderem Vorteil, weil entsprechende Verbindungen in nachgeschalteten Schritten negative Effekte haben könnten. Beispielsweise neigt das Diolefin Butadien bei den in der nachgeschalteten trenntechnischen Bearbeitung verwendeten Trennbedingungen zur Polymerisierung. Dies wird durch die Hydrierung verhindert.

Das erfindungsgemäße Verfahren sieht vorteilhafterweise ferner vor, stromauf der Druckwechseladsorption und stromauf der oben erwähnten ersten Verdichtung eine Wasserwäsche vorzunehmen. Die Bereitstellung des Produktgemischs umfasst daher vorteilhafterweise eine derartige Wasserwäsche. Eine derartige Wasserwäsche dient insbesondere zur Entfernung schwerer Kohlenwasserstoffe bzw. ölartiger Komponenten, wie sie auch der oxidativen Kopplung von Methan als Nebenprodukte gebildet werden können. Entsprechende Wasserwäscheverfahren sind aus dem Bereich der Dampfspaltverfahren bekannt. Eine Wasserwäsche wird im Englischen auch als "Scrubbing" bezeichnet. Eine Wasserwäsche stromauf der ersten Verdichtung und der Druckwechseladsorption ist besonders vorteilhaft, weil auf diese Weise entsprechende Komponenten in der Druckwechseladsorption nicht stören können.

Im Rahmen der vorliegenden Erfindung werden vorteilhafterweise unter Verwendung der Druckwechseladsorption und/oder zumindest eines der weiteren Aufbereitungsschritte ein oder mehrere Fluidströme gebildet, die zur oxidativen Kopplung von Methan zurückgeführt werden. Entsprechende Fluidströme können auch aufbereitet werden, beispielsweise kann Kohlendioxid mit Wasserstoff einer Methanisierung unterworfen werden. Abgetrenntes Methan oder in einer entsprechenden Methanisierung gebildetes Methan kann in die oxidative Kopplung von Methan zurückgeführt und dort als Einsatz verwendet werden. Abgetrenntes Ethan und höhere Paraffine können beispielsweise einem postkatalytischen Dampfspalten unterworfen werden.

Die Erfindung bezieht sich auch auf eine Anlage zur Erzeugung und Verarbeitung von Produkten der oxidativen Kopplung von Methan, die dafür eingerichtet ist, unter Verwendung eines Verfahrens zur oxidativen Kopplung von Methan ein Gasgemisch bereitzustellen, das zumindest Ethylen, Ethan und Methan sowie niedriger als Methan und höher als Ethan siedende Verbindungen enthält. Diese zeichnet sich erfindungsgemäß dadurch aus, dass sie dafür eingerichtet ist, aus dem Gasgemisch unter Verwendung einer Druckwechseladsorption einen Teil des Methans und zumindest ein Teil der niedriger als Methan siedenden Komponenten zu entfernen, und dass sie dafür eingerichtet ist, das Gasgemisch anschließend weiteren Aufbereitungsschritten zu unterwerfen, wobei die Anlage ferner dafür eingerichtet ist, die Druckwechseladsorption unter Verwendung eines unteratmosphärischen Desorptionsdruckniveaus durchzuführen.

Eine entsprechende Anlage ist insbesondere zur Ausführung eines Verfahrens eingerichtet, wie es zuvor erläutert wurde. Auf die entsprechenden Erläuterungen wird daher ausdrücklich verwiesen.

Die Erfindung wird in bevorzugten Ausgestaltungen unter Bezugnahme auf die beigefügten Zeichnungen näher erläutert.

### Kurze Beschreibung der Zeichnung

Figur 1 veranschaulicht einen Prozess gemäß einer Ausführungsform der Erfindung in Form eines schematischen Ablaufplans.
Figur 2 veranschaulicht einen Prozess gemäß einer Ausführungsform der Erfindung in Form eines schematischen Ablaufplans.

### Ausführliche Beschreibung der Zeichnung

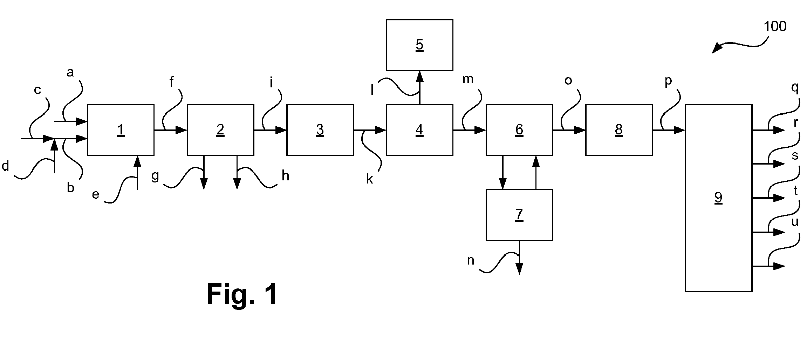

In Figur 1 ist ein Prozess gemäß einer besonders bevorzugten Ausführungsform der Erfindung in Form eines schematischen Ablaufplans veranschaulicht und insgesamt mit 100 bezeichnet.

Der Prozess 100 umfasst ein Verfahren zur oxidativen Kopplung von Methan, das hier insgesamt mit 1 bezeichnet ist. Wie erwähnt, kann ein entsprechendes Verfahren in einem geeigneten Reaktor durchgeführt werden, der insbesondere auch zusätzlich zum postkatalytischen Dampfspalten ("Post Bed Cracking") eingerichtet, und mit einem Quenchkühler zur Abkühlung des Produktgemisches verbunden sein kann. Zu Details sei auf einschlägige Fachliteratur verwiesen.

Dem Verfahren zur oxidativen Kopplung von Methan 1 werden ein sauerstoffreicher Strom a und ein methanreicher Strom b zugeführt. Der methanreiche Strom b kann unter Verwendung eines extern bereitgestellten Methanstroms c und eines oder mehrerer rückgeführter Methanströme d gebildet werden. Der extern bereitgestellte Methanstrom c kann insbesondere auch bestimmte Mengen anderer Komponenten außer Methan, die beispielsweise in Erdgas oder aufgereinigten Erdgasfraktionen üblich sind, enthalten Die Rückführung eines Methanstroms wird unten erläutert. Ein ebenfalls rückgeführter, ethanreicher Strom e kann beispielsweise in einen postkatalytischen Dampfspaltschritt geführt werden.

Unter Verwendung des Verfahrens zur oxidativen Kopplung von Methan 1 wird ein Gasgemisch ("Produktgemisch") gebildet, das zumindest Methan, Ethan und Ethylen, niedriger als Methan und höher als Ethan siedende Verbindungen enthält, insbesondere auch Wasser und Kohlendioxid. Dieses Produktgemisch kann als Strom f einer Wasserwäsche 2 zugeführt werden. In der Wasserwäsche 2 können unter Einsatz von Waschwasser schwere Komponenten aus dem Produktgemisch des Stroms f ausgewaschen und in Form eines Stroms g abgezogen werden. Ein hierbei ebenfalls anfallender Wasserstrom ist mit h bezeichnet.

Das entsprechend von schweren Komponenten befreite Produktgemisch kann in Form eines Stroms i einer ersten Verdichtung 3 zugeführt werden, wo es im Rahmen einer Ausführungsform der vorliegenden Erfindung auf ein geeignetes Druckniveau verdichtet wird. Das verdichtete Produktgemisch kann, wie hier in Form eines Stroms k veranschaulicht, anschließend einer Druckwechseladsorption 4 zugeführt werden. In der Druckwechseladsorption 4 kann das Produktgemisch des Stroms k an Methan und an niedriger als Methan siedenden Verbindungen abgereichert werden. Ein hierbei erhaltenes Gasgemisch, das beispielsweise außer Methan auch Kohlenmonoxid, Wasserstoff und Inertgase enthalten kann, kann in Form eines Stroms l einer Aufbereitung 5 beliebiger Art, beispielsweise einer Methanisierung oder einem Trennschritt, unterworfen werden. Bei Bedarf kann ein entsprechendes Gasgemisch hierbei in geeigneter Weise, beispielsweise durch Entfernung, Zugabe und/oder Umsetzung von Komponenten, konditioniert werden, so dass es sich für die Rückführung in das Verfahren zur oxidativen Kopplung von Methan 1 eignet. In die Aufbereitung 5 können neben dem Strom l auch weitere Ströme, wie hier nicht gesondert veranschaulicht, geführt werden.

Im Rahmen der in Figur 1 gezeigten Ausführungsform der Erfindung werden in der Druckwechseladsorption 4 ein überatmosphärisches Adsorptionsdruckniveau und ein unteratmosphärisches Desorptionsdruckniveau verwendet. Ein an Methan und tiefer siedenden Komponenten ab- und an höher an Methan siedenden Komponenten angereichertes Gasgemisch wird in Form eines Stroms m entnommen. Das Gasgemisch ist zwar an den in der Druckwechseladsorption 4 nicht adsorbierten Komponenten abgereichert, kann aber insbesondere noch beträchtliche Mengen an Methan enthalten. Die Abtrennung von Methan ist jedoch aufgrund des mehrfach geschilderten Betriebs der Druckwechseladsorption 4, d.h. der Entnahme des Stroms m auf dem unteratmosphärischen Desorptionsdruckniveau, deutlich effizienter, so dass stromab der Druckwechseladsorption 4 nur noch deutlich geringere Gasvolumina bearbeitet werden müssen.

Das in der Druckwechseladsorption 4 insbesondere an Methan abgereicherte Gasgemisch kann in Form des Stroms m einer zweiten Verdichtung 6 unterworfen werden. Im Zuge oder nach dieser zweiten Verdichtung 6 kann eine Sauergasentfernung 7 erfolgen, indem ein entsprechendes Gasgemisch auf einer Zwischenstufe der zweiten Verdichtung 6 entzogen, einem Waschschritt in der Sauergasentfernung 7 unterworfen, und anschließend der zweiten Verdichtung 6 auf der Zwischenstufe erneut zugeführt wird. Wie erwähnt, können zur Sauergasentfernung bekannte Gaswäscheverfahren, insbesondere Aminwäschen und Laugenwäschen, zum Einsatz kommen. In der Sauergasentfernung gebildetes Kohlendioxid kann in Form eines Stroms n abgezogen und in einer als Methanisierung ausgebildeten Aufbereitung 5 eingesetzt oder anderweitig verwendet werden.

Das verdichtete und an Sauergasen befreite Gasgemisch kann in Form eines Stroms o einer Trocknung und Vorkühlung 8 unterworfen werden, in der Wasser, beispielsweise adsorptiv, aus dem Produktgemisch des Stroms o abgetrennt werden kann. Auf diese Weise kann das Produktgemisch anschließend in Form eines Stroms p in im Wesentlichen wasserfreiem Zustand einer Tieftemperaturtrennung 9 zugeführt werden, ohne dass Wasser ausfrieren kann. Ein Hydrierungsschritt (nicht gesondert veranschaulicht) kann stromauf der eigentlichen Tieftemperaturtrennung 9 vorgesehen sein, in dem in dem Produktgemisch des Stroms p enthaltene Acetylene und/oder Diolefine zu den entsprechenden Olefinen hydriert werden können.

Die Tieftemperaturtrennung 9 kann in beliebiger Weise vorgenommen werden. In der Tieftemperaturtrennung 9 können beispielsweise aus dem eingangs zitierten Stand der Technik bekannte Demethanizer, Deethanizer, Depropanizer, C2-Splitter, C3-Splitter und dergleichen eingesetzt werden. Auch die Verwendung absorptiv arbeitender Verfahren, bei denen bestimmte Komponenten mittels einer Absorptionsflüssigkeit ausgewaschen werden, und die anschließend regeneriert werden, ist möglich.

In der Tieftemperaturtrennung 9 können eine Reihe von Reingasen oder Gasgemischen gebildet werden, die hier nur exemplarisch in Form der Ströme q bis u veranschaulicht sind. Diese umfassen beispielsweise Methan, Strom q, das zumindest zum Teil in Form des bereits erwähnten Stroms d in die oxidative Kopplung von Methan 1 zurückgeführt werden kann, Ethan, Strom r, und/oder Propan, Strom t, das zumindest zum Teil in Form des bereits erwähnten Stroms e zum postkatalytischen Dampfspalten zurückgeführt werden kann, Ethylen, Strom s, und gegebenenfalls Propylen, Strom u das zumindest zum Teil als Produkt ausgeführt werden kann, sowie höhere Kohlenwasserstoffe wie mit mindestens drei Kohlenstoffatomen.

Durch die Abreicherung an Methan in der Druckwechseladsorption 4 ist es möglich, die in der Tieftemperaturtrennung 9 zu bearbeitenden Gasvolumina deutlich zu verringern. Hierdurch kann der Anlagenteil nach der Druckwechseladsorption 4 inklusive dem "kalten Teil" einer entsprechenden Anlage deutlich kleiner dimensioniert werden, was beispielsweise den Bedarf an Kältemitteln reduziert und die Erstellungskosten durch Einsparung tieftemperaturbeständiger Materialien verringert. Aufgrund der relativ geringen Anteile an höheren Kohlenwasserstoffen gegenüber dem Hauptbestandteil Methan in entsprechenden Gasgemischen besteht die volumenmäßig herausforderndste Trennaufgabe in der Abtrennung des Methans. Diese wird durch den Einsatz der Druckwechseladsorption mit Gasentnahme auf unteratmosphärischem Druckniveau realisiert. Die höher als Methan siedenden Komponenten können bei entsprechender Reduzierung der Methanmenge in vergleichsweise klein dimensionierten Einheiten voneinander getrennt werden.

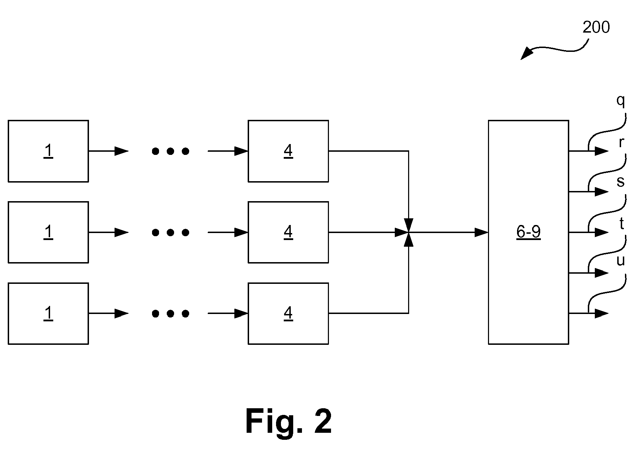

In Figur 2 ist ein Prozess gemäß einer Ausführungsform der Erfindung veranschaulicht und insgesamt mit 200 bezeichnet. Der Prozess 200 zeichnet sich durch eine Parallelisierung der Verfahrensschritte 1 bis 5 gemäß Figur 1 aus, die hier nochmals stark vereinfacht veranschaulicht sind. Nicht explizit veranschaulichte Zwischenschritte sind durch Auslassungspunkte symbolisiert.

Wie ersichtlich, erfolgt im dargestellten Beispiel die die Parallelisierung in dem Prozess 200 zumindest bis zur Druckwechseladsorption 4. Die zweite Verdichtung 6 und die Sauergasentfernung 7 sowie die Vorkühlung und Trocknung 8 können, wie hier dargestellt, bereits gemeinsam durchgeführt werden. Durch die Parallelisierung vorzugsweise bis zur Druckwechseladsorption kann eine effektive Umsetzung durch oxidative Kopplung von Methan sowie eine effektive Trennung erreicht werden, ohne an Baubarkeitsgrenzen zu stoßen. Die volumenintensiven Trennschritte bis zur Druckwechseladsorption sind parallelisiert ausgeführt, wenn die sinnvolle Baubarkeitsgrenze für eine einstängige Verfahren bzw. Verfahrensschritte erreicht wird, sich ein Kostenvorteil abzeichnet oder die Anlageneffizienz dadurch gesteigert werden kann. Diese Kriterien der Parallelizierung können auch für eine mehrsträngige Verfahren bzw. Verfahrensschritte angewendet werden. Die Prozessschritte 6 bis 9 können Gasgemische aus einer Vielzahl paralleler Verfahren bzw. Verfahrensschritte bearbeiten, da der Gehalt an höher als Methan siedenden Komponenten gering ist und durch die erfindungsgemäß auf dem unteratmosphärischen dritten Druckniveau betriebene Druckwechseladsorption 4 Methan und niedriger als Methan siedende Komponenten zu einem großen Teil entfernt wurden.

## Patentansprüche

1. Prozess (100, 200) zur Erzeugung und Verarbeitung von Produkten der oxidativen Kopplung von Methan, bei dem unter Verwendung eines Verfahrens zur oxidativen Kopplung von Methan (1) ein Gasgemisch bereitgestellt wird, das zumindest Ethylen, Ethan und Methan sowie niedriger als Methan und höher als Ethan siedende Verbindungen enthält, **dadurch gekennzeichnet, dass** aus dem Gasgemisch unter Verwendung einer Druckwechseladsorption (4) ein Teil des Methans und zumindest ein Teil der niedriger als Methan siedenden Komponenten entfernt werden, und dass das Gasgemisch anschließend weiteren Aufbereitungsschritten unterworfen wird, wobei die Druckwechseladsorption (4) die Verwendung eines unteratmosphärischen Desorptionsdruckniveaus umfasst.

2. Prozess (100, 200) nach Anspruch 1, bei dem das Desorptionsdruckniveau bei 200 bis 800 mbar, insbesondere bei 350 bis 500 mbar, liegt.

3. Prozess (100, 200) nach einem der vorstehenden Ansprüche, bei dem die Druckwechseladsorption (4) die Verwendung eines Adsorptionsdruckniveaus von 10 bis 30 bar, insbesondere von 13 bis 17 bar, umfasst.

4. Prozess (100, 200) nach einem der vorstehenden Ansprüche, bei dem unter Verwendung der Druckwechseladsorption (4) aus dem Gasgemisch 70 bis 90 Molprozent, insbesondere 80 bis 85 Molprozent, des Methans entfernt werden.

5. Prozess (100, 200) nach einem der vorstehenden Ansprüche, bei dem unter Verwendung mehrerer Einrichtungen, die zur oxidativen Kopplung von Methan (1) eingerichtet sind, jeweils ein Gasgemisch bereitgestellt wird, das zumindest Ethylen, Ethan und Methan sowie niedriger als Methan und höher als Ethan siedende Verbindungen enthält, wobei aus den Gasgemischen parallel zueinander in wenigstens zwei Einrichtungen, die zur Druckwechseladsorption (4) eingerichtet sind, ein Teil des Methans und zumindest ein Teil der niedriger als Methan siedenden Komponenten entfernt werden, und wobei die Gasgemische anschließend vereinigt und zumindest einem der weiteren Aufbereitungsschritte unterworfen werden.

6. Verfahren nach einem der vorstehenden Ansprüche, bei dem die weiteren Aufbereitungsschritte eine Tieftemperaturtrennung (9) umfassen.

7. Verfahren nach einem der vorstehenden Ansprüche, bei dem die weiteren Aufbereitungsschritte wenigstens einen absorptiven Schritt umfassen.

8. Verfahren nach einem der vorstehenden Ansprüche, bei dem unter Verwendung der Druckwechseladsorption (4) und/oder zumindest eines der weiteren Aufbereitungsschritte ein oder mehrere Fluidströme gebildet werden, die zur oxidativen Kopplung von Methan (1) zurückgeführt werden.

9. Anlage zur Erzeugung und Verarbeitung von Produkten der oxidativen Kopplung von Methan, die dafür eingerichtet ist, unter Verwendung eines Verfahrens zur oxidativen Kopplung von Methan (1) ein Gasgemisch bereitzustellen, das zumindest Ethylen, Ethan und Methan sowie niedriger als Methan und höher als Ethan siedende Verbindungen enthält, **dadurch gekennzeichnet, dass** die Anlage dafür eingerichtet ist, aus dem Gasgemisch unter Verwendung einer Druckwechseladsorption (4) ein Teil des Methans und zumindest ein Teil der niedriger als Methan siedenden Komponenten zu entfernen, und dass die Anlage dafür eingerichtet ist, das Gasgemisch anschließend weiteren Aufbereitungsschritten zu unterwerfen, wobei die Anlage ferner dafür eingerichtet ist, die Druckwechseladsorption (4) unter Verwendung eines unteratmosphärischen Desorptionsdruckniveaus durchzuführen.

10. Anlage nach Anspruch 9, die zur Durchführung eines Verfahrens nach einem der Ansprüche 1 bis 8 eingerichtet ist.
